(19) Europäisches Patentamt
European Patent Office
Office européen des brevets

(11) Veröffentlichungsnummer: **0 021 382**
**B1**

(12) # EUROPÄISCHE PATENTSCHRIFT

(45) Veröffentlichungstag der Patentschrift: **28.04.82**

(21) Anmeldenummer: **80103500.7**

(22) Anmeldetag: **23.06.80**

(51) Int. Cl.³: **C 07 C 69/74**, C 07 C 69/743, C 07 C 69/747, C 07 C121/46, C 07 C121/48, C 07 C147/04

(54) **Verfahren zur Herstellung von Cyclopropan-carbonsäurederivaten sowie neue Zwischenprodukte hierfür und Verfahren zu deren Herstellung.**

(30) Priorität: **02.07.79 DE 2926671**

(43) Veröffentlichungstag der Anmeldung: **07.01.81 (Patentblatt 81/01)**

(45) Bekanntmachung des Hinweises auf die Patenterteilung: **28.04.82 Patentblatt 82/17**

(84) Benannte Vertragsstaaten: **BE CH DE FR GB IT LI NL**

(56) Entgegenhaltungen:
**DE - A - 2 243 371**
**DE - A1 - 2 500 265**
**DE - A1 - 2 650 534**
**DE - B - 1 289 046**
**DE - B - 1 668 603**
**US - A - 3 578 717**

(73) Patentinhaber: **BAYER AG**
**Zentralbereich Patente, Marken und Lizenzen**
**D-5090 Leverkusen 1, Bayerwerk (DE)**

(72) Erfinder: **Hanack, Michael, Prof. Dr.**
**Panoramastrasse 39**
**D-7400 Tübingen 7 (DE)**
Erfinder: **Stoll, Theodor, Dipl.-Chem.**
**Schneekoppestrasse 4**
**D-7030 Böblingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 021 382 B1

Imprimerie Jouve, 18, rue St-Denis, 75001 Paris, France

# Verfahren zur Herstellung von Cyclopropan-carbonsäurederivaten sowie neue Zwischenprodukte hierfür und Verfahren zu deren Herstellung

Die Erfindung betrifft ein neues Verfahren zur Herstellung von Cyclopropan-carbonsäure-derivaten sowie Perfluoralkylsulfone als neue Zwischenprodukte hierfür und ein neues Verfahren zu deren Herstellung.

Es ist bereits bekannt, Perfluoralkylsulfone durch Addition von Perfluoralkansulfinsäuren an Doppelbindungen, die durch CN, Carboxyl oder Carbonamidresten aktiviert sind, herzustellen (DE-OS 2 243 371). Es war jedoch nichts über eine Addition von Perfluoralkansulfinsäuren an Diene bekannt.

Es ist bereits bekannt, daß man 2,2-Dimethyl-3-(2-methyl-1-propenyl)-cyclopropan-1-carbonsäure-derivate erhält, wenn man Aryl-(3-methyl-2-butenyl)-sulfone mit β,β-Dimethyl-acrylsäure-derivaten in Gegenwart von Basen umsetzt (vgl. DE-AS 1 289 046).

Zur Durchführung dieses Verfahrens werden, sehr starke Basen und wasserfreie Lösungsmittel benötigt. Die Ausbeuten sind, insbesondere bei der Herstellung der als Ausgangsverbindungen benötigten Aryl-(3-methyl-2-butenyl) sulfone, unbefriedigend.

Gegenstand der vorliegenden Erfindung sind :

1. Ein Verfahren zur Herstellung von Cyclopropan-1-carbonsäure-derivaten der Formel I

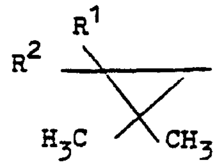

(I)

in welcher

Z für Alkoxycarbonyl oder Cyano steht und

$R^1$ für Wasserstoff oder für Alkenyl steht und

$R^2$ für Wasserstoff oder gemeinsam mit $R^1$ für Cycloalkyl oder Cycloalkenyl, and die gegebenenfalls ein Benzolring annelliert sein kann, steht.

dadurch gekennzeichnet, daß man Perfluoralkylsulfone der Formel II

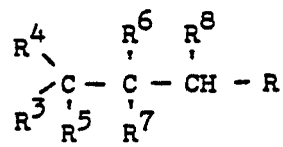

(II)

in welcher

R für den Rest $-SO_2-R_F$,

$R^3$ für Wasserstoff oder Alkyl steht,

$R^4$ für Alkyl steht oder zusammen mit $R^8$ eine Alkendiylbrücke bildet, deren Doppelbindung gegebenenfalls an einen Benzolring annelliert ist,

$R^5$ und $R^6$ entweder für Wasserstoff stehen oder gemeinsam eine Doppelbindung bilden.

$R^7$ für Wasserstoff oder Alkyl steht,

$R^8$ für Wasserstoff oder Alkyl steht und

$R_F$ für Perfluoralkyl steht,

mit der Maßgabe, daß $R^5$ und $R^6$ entweder eine Doppelbindung bilden oder $R^8$ und $R^4$ eine Alkendiylbrücke, deren Doppelbindung gegebenenfalls an einen Benzolring annelliert ist, bilden,

mit β,β-Dimethyl-acrylsäure-derivaten der Formel III

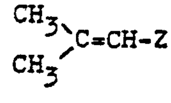

(III)

in welcher

Z die oben angegebene Bedeutung hat,

in Gegenwart von Basen und gegebenenfalls in Gegenwart von Verdünnungsmitteln bei Temperaturen zwischen − 20 und + 30 °C umsetzt ;

2. Neue Perfluoralkyl-sulfone der Formel II

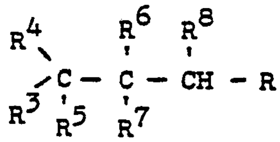

(II)

in welcher R, $R^3$-$R^8$ die oben angegebene Bedeutung besitzen.

3. Ein Verfahren zur Herstellung von Perfluoralkylsulfonen der Formel II (oben), dadurch gekennzeichnet, daß man Perfluoralkan-sulfinsäuren der Formel IV

$$R_F — SO_2H \qquad (IV)$$

in welcher

$R_F$ für Perfluoralkyl steht,

mit Dienen der Formel V

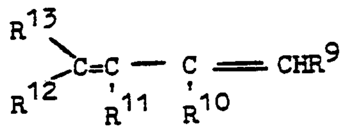

(V)

in welcher

$R^9$-$R^{13}$ für Wasserstoff oder Alkyl stehen, wobei

$R^9$ gemeinsam mit $R^{13}$ für eine Alkylenbrücke stehen kann, an die gegebenenfalls ein Cycloalkenring annelliert ist, gegebenenfalls in Gegenwart von Verdünnungsmitteln bei Temperaturen zwischen − 20 und + 20 °C umsetzt.

Es ist als überraschend anzusehen, daß durch die Verwendung von Perfluoralkylsulfonen der Formel (II) Cyclopropan-1-carbonsäurederivate der Formel (I) in guten Ausbeuten und in hoher Reinheit hergestellt werden können, da bei der bekannten Synthesemethode, bei welcher entsprechende Arylsulfone eingesetzt werden, nur mäßige, schwer reproduzierbare Ausbeuten zu erzielen sind.

Weiter ist es als überraschend zu bezeichnen, daß die neuen Perfluoralkylsulfone der Formel (II) durch eine einfach durchzuführende Additionsreaktion von Perfluoralkansulfinsäuren an Diene in hohen Ausbeuten synthetisiert werden können. Die Kombination der beiden neuen Verfahren eröffnet eine technisch vorteilhafte Möglichkeit zur Herstellung von Derivaten der Cyclopropancarbonsäure, welche Zwischenprodukte für Pyrethroide sind.

Verwendet man beispielsweise als Ausgangsstoffe bei Verfahren (3) Perfluormethansulfinsäure und Isopren und bei der mit dem hierbei gebildeten (3-Methyl-2-butenyl)-trifluormethyl-sulfon durchzuführenden Umsetzung nach Verfahren (1) als weitere Reaktionskomponente β,β-Dimethyl-acrylsäurenitril, so können die Reaktionsabläufe durch folgendes Formelschema skizziert werden :

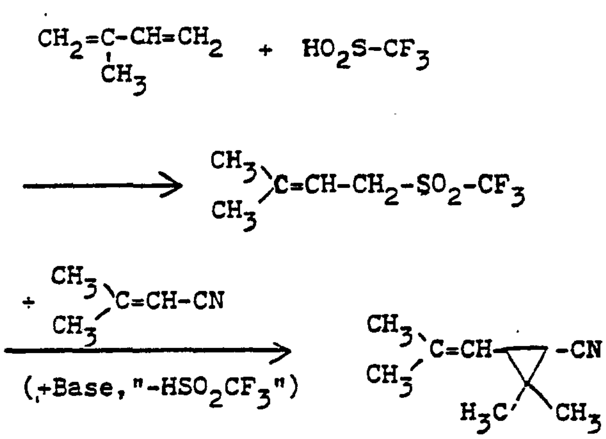


Das unter (1) dargelegte neue Verfahren (« Verfahren (1) ») wird vorzugsweise unter Verwendung von Verdünnungsmitteln durchgeführt. Als solche kommen praktisch alle organischen Lösungsmittel in Frage. Besonders geeignet sind Alkohole, wie z.B. Methanol, Ethanol, n- und iso-Propanol, sowie insbesondere aprotisch polare Lösungsmittel. Hierzu gehören beispielsweise Ether, wie z.B. Diethylether, Glycoldimethylether, Tetrahydrofuran und Dioxan, Nitrile, wie z.B. Acetonitril und Propionitril, Carbonsäureamide, wie z.B. Dimethylformamid und Dimethylacetamid, Sulfoxide und Sulfone, wie z.B. Dimethylsulfoxid und Tetramethylsulfon, sowie Phosphorsäureamide, wie z.B. Hexamethylphosphorsäuretriamid. Tetrahydrofuran wird als Lösungsmittel besonders bevorzugt.

Bei Verfahren (1) können die in der organisch-chemischen Synthese üblichen Basen verwendet werden. Hierzu gehören insbesondere Alkali- und Erdalkali-hydroxide, wie z.B. Natrium, Kalium- und Calcium-hydroxid, Alkalialkoholate, wie z.B. Natrium- und Kalium-methylat, Natrium- und Kalium-ethylat, Natrium- und Xalium-tert.-butylat, ferner aliphatische, aromatische und heterocyclische Amine, wie z.B. Triethylamin, Ethyldiisopropylamin, N,N-Dimethyl-benzylamin, N,N-Dimethyl-anilin, Pyridin, N-Methylpiperidin und Diazabicyclononan.

Alkoholate werden als Basen bei Verfahren (1) besonders bevorzugt. Die Reaktionstemperatur wird bei Verfahren (1) zwischen – 20 und + 30 °C, vorzugsweise zwischen – 15 und + 25 °C gehalten. Das Verfahren wird im allgemeinen bei Normaldruck durchgeführt.

Auf 1 Mol Perfluoralkyl-sulfon der Formel (II) setzt man im allgemeinen zwischen 1 und 5 Mol, vorzugsweise zwischen 1,5 und 4 Mol, β,β-Dimethyl-acrylsäure-derivat der Formel (III) ein.

In einer bevorzugten Ausführungsform von Verfahren (1) wird die Hauptmenge der einzusetzenden Base in einem der oben genannten Verdünnungsmittel unter Inertgasatmosphäre, z.B. unter Stickstoff, vorgelegt und auf 0 bis – 15 °C abgekühlt. Dann werden nacheinander das Acrylsäurederivat der Formel (III) und das Sulfon der Formel (II) langsam zugetropft. Das komplette Reaktionsgemisch wird jeweils mehrere Stunden bei 0 bis – 15 °C, dann bei – 5 bis + 5 °C und schließlich bei + 15 bis + 25 °C gerührt. Dann kühlt man das Gemisch wieder auf die Anfangstemperatur ab, gibt etwa 1/10 der anfangs eingesetzten Basenmenge dazu und rührt wie oben angegeben bei verschiedenen Temperaturen.

Dieser Vorgang wird gegebenenfalls mehrfach wiederholt. Die Aufarbeitung kann nach üblichen Methoden durchgeführt werden, beispielsweise, indem man das Reaktionsgemisch in Wasser gießt, mit einem mit Wasser nicht mischbaren Lösungsmittel, wie z.B. Diethylether, extrahiert, die nicht-wässrige Phase trocknet und nach Filtration das Lösungsmittel abdestilliert. Das hierbei zurückbleibende Produkt kann durch Vakuum-destillation gereinigt werden. Zur Charakterisierung dienen das IR- und das NMR-Spektrum.

Die neuen Perfluoralkylsulfone, welche als Ausgangsstoffe für das erfindungsgemäße Verfahren (1) zu verwenden sind, sind durch Formel (II) definiert. Bevorzugt sind Perfluoralkylsulfone der Formel VI

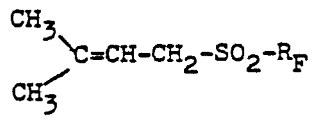

(VI)

in welcher

$R_F$ für Perfluoralkyl mit 1-8 C-Atomen steht.

Als Beispiele für die Verbindungen der Formel (II) seien Perfluormethyl-(3-methyl-2-butenyl)-sulfon, Perfluorbutyl-(3-methyl-2-butenyl)-sulfon und Perfluoroctyl-(3-methyl-2-butenyl)-sulfon genannt.

Man erhält die neuen Perfluoralkylsulfone der Formel (II) nach dem oben unter (3) dargelegten Verfahren (« Verfahren (3) ») durch Umsetzung von Perfluoralkansulfinsäuren mit Dienen.

Die hierbei als Ausgangsstoffe einzusetzenden Perfluoralkansulfinsäuren sind durch Formel (IV) definiert.

Vorzugsweise stehen darin

$R_F$ für Perfluoralkyl mit 1 bis 8 Kohlenstoffatomen.

Als Beispiele für die Ausgangsstoffe der Formel (IV) seien Perfluormethansulfinsäure, Perfluorbutansulfinsäure und Perfluoroctansulfinsäure genannt.

Perfluoralkansulfinsäuren sind bekannte Verbindungen (vgl. Liebigs Ann. Chem. *1973,* 33).

Die weiter als Ausgangsstoffe zu verwendenden Diene sind durch Formel (V) definiert. Vorzugsweise stehen darin

$R^9$ für Wasserstoff, Methyl oder zusammen mit $R^{13}$ für Methylen oder Äthylen,

$R^{10}$ und $R^{11}$ für Wasserstoff oder Methyl, und

$R^{12}$ und $R^{13}$ für Wasserstoff oder Methyl, wobei $R^{12}$ und $R^{11}$ gemeinsam mit den an sie angrenzenden C-Atomen einen Benzolring bilden können.

Als Beispiele für die Ausgangsverbindungen der Formel (V) seien genannt :

1,3-Butadien, 1,3-Pentadien, 2,4-Hexadien, 2,3-Dimethyl-1,3-butadien, 2-Methyl-1,3-butadien, Cyclopentadien, Inden und Cyclohexadien.

Verfahren (3) kann ohne Verwendung von Lösungsmitteln durchgeführt werden. Es können jedoch auch die oben für Verfahren (1) angegebenen polaren Lösungsmittel wie auch unpolare oder geringfügig polare Solventien, wie aliphatische oder aromatische, gegebenenfalls halogenierte, Kohlenwasserstoffe, wie z.B. Pentan, Hexan, Heptan, Toluol, Chlorbenzol, Methylenchlorid, Chloroform und 1,2-Dichlorethan verwendet werden.

Die Reaktionstemperatur wird bei Verfahren (3) zwischen – 20 und + 20 °C, vorzugsweise zwischen – 10 und + 10 °C gehalten. Das Verfahren wird im allgemeinen bei Normaldruck durchgeführt.

Je Mol Perfluoralkansulfinsäure der Formel (IV) werden bei Verfahren (3) 1 bis 5 Mol, vorzugsweise 1,5 bis 3 Mol Dien der Formel (V) eingesetzt.

In einer bevorzugten Ausführungsform von Verfahren (3) wird die Perfluoralkansulfinsäure vorgelegt und mit Dien der Formel (V) im Überschuß tropfenweise versetzt. Die Aufarbeitung erfolgt auf übliche

Weise : man gibt das Reaktionsgemisch in Eiswasser, neutralisiert und extrahiert mit einem mit Wasser nicht mischbaren Lösungsmittel, wie z.B. Diethylether. Von der nichtwässrigen Phase wird nach Trocknen und Filtrieren das Lösungsmittel abdestilliert. Das als Rückstand verbleibende Produkt kann durch Vakuumdestillation gereinigt werden. Es wird durch IR- und NMR-Spektrum charakterisiert.

Die bei Verfahren (1) neben den neuen Sulfonen der Formel (II) als weitere Ausgangsstoffe einzusetzenden β,β-Dimethyl-acrylsäure-derivate sind durch Formel (III) definiert. Vorzugsweise steht darin

Z für Alkoxycarbonyl mit 1 bis 4 Kohlenstoffatomen im Alkoxyrest oder für Cyano.

Als Beispiele für die Ausgangsstoffe der Formel (III) seien

β,β-Dimethyl-acrylsäure-methylester, -ethylester, -n-propylester, -iso-propylester, -n-butylester, -isobutylester, -sek.-butylester und tert.-butylester sowie β,β-Dimethyl-acrylsäurenitril genannt. Der Methylester und der Ethylester werden besonders bevorzugt.

β,β-Dimethyl-acrylsäure-derivate der Formel (III) sind bekannt (vgl. DE-AS 1 289 046).

Die nach dem erfindungsgemäßen Verfahren herzustellenden Cyclopropan-1-carbonsäure-derivate der Formel (I) können als Zwischenprodukte zur Herstellung von insektizid und akarizid wirksamen Pyrethroiden verwendet werden (vgl. DE-AS 1 289 046 und DE-OS 2 605 828).

Beispiel 1

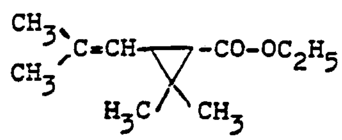

22 g β,β-Dimethyl-acrylsäure-ethylester werden zu einer Mischung aus 10 g Kalium-tert.-butylat und 100 ml Tetrahydrofuran, welche man auf – 10 °C abgekühlt und mit Stickstoff begast hat, tropfenweise gegeben. Anschließend wird eine Lösung von 17 g Perfluorbutyl-(3-methyl-2-butenyl) sulfon in 20 ml Tetrahydrofuran sehr langsam unter kräftigem Rühren zugetropft. Das Reaktionsgemisch wird noch 12 Stunden bei – 10 °C, noch einmal 12 Stunden bei 0 °C und 24 Stunden bei Zimmertemperatur gerührt. Es wird dann wieder auf – 10 °C abgekühlt und eine Lösung von 1 g Kaliumtert.-butylat in 20 ml Tetrahydrofuran zugetropft. Die Temperaturerhöhung wird wie oben beschrieben durchgeführt. Dieser Vorgang wird noch zweimal wiederholt. Das Gemisch wird in Wasser gegossen und mit Diethylether eine Extraktion durchgeführt. Der Etherextrakt wird über Magnesiumsulfat getrocknet, filtriert und eingeengt. Nach Destillation unter vermindertem Druck erhält man 2,2-Dimethyl-3-(2-methyl-1-propenyl)-cyclopropan-1-carbonsäure-ethylester vom Siedepunkt 70 °C/5 Torr als farblose Flüssigkeit in einer Ausbeute von 60 % der Theorie.

Charakterisierung durch die Spektren :

$^1$H-NMR ($CCl_4$) : δ = 1,2 (m, 10H, Methylprotonen und Cyclopropylproton), 1,72 (s, 7H, Methylprotonen und Cyclopropylproton), 4,08 (q, 2H, Methylenprotonen, 4,87 (d, 1H, Vinylproton) ppm.

IR (Film) : 3 010 (olefin. C-H), 1 735 (Estercarbonyl), 1 670 (Doppelbindung), 1 455 ($—CH_3$), 1 390 ($—CH_3$) cm$^{-1}$.

Beispiel 2

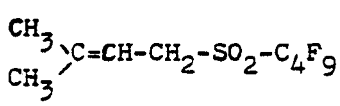

25 g Perfluorbutansulfinsäure (0,088 mol) werden in einem 100 ml Dreihalskolben vorgelegt und auf 0 °C abgekühlt. Dann wird frisch destilliertes Isopren im Überschuß zugetropft, wobei eine Tropfgeschwindigkeit von 1 Tropfen pro Minute eingehalten werden sollte. Nach dem Zutropfen wird die Lösung sofort in Eiswasser gegossen, mit $NaHCO_3$ neutralisiert und ausgeethert. Nach dem Trocknen mit $MgSO_4$ wird abfiltriert, der Äther am Rotationsverdampfer abgezogen und der Rückstand destilliert. Man erhält Perfluorbutyl-(3-methyl-2-butenyl) sulfon vom Siedepunkt 41 °C/0,1 Torr als gelbstichige Flüssigkeit in einer Ausbeute von 60 bis 70 % der Theorie.

Charakterisierung durch die Spektren :

$^1$H-NMR ($CCl_4$) : δ = 1.79 (S, 3H, Methylprotonen),
1.87 (s, 3H, Methylprotonen),
4.05 (d, 2H, Methylenprotonen),
5.27 (t, 1H, Vinylproton) ppm.

IR (Film) : 3 000 (olefin. C-H), 2 930 (aliphat. C-H),

1 660 (Doppelbindung), 1 450 ($-CH_3$), 1 370, 1 145 ($SO_2$), 1 200-1 250 ($C_4F_9$) $cm^{-1}$.

Analog können hergestellt werden :

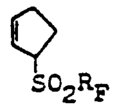

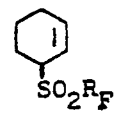

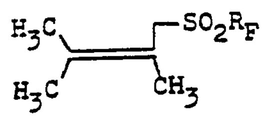

**Ansprüche**

1. Verfahren zur Herstellung von Cyclopropan-1-carbonsäure-derivaten der Formel I

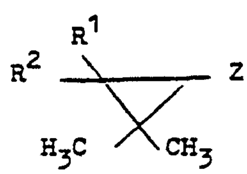

(I)

in welcher

Z für Alkoxycarbonyl oder Cyano steht und

$R^1$ für Wasserstoff oder für Alkenyl steht und

$R^2$ für Wasserstoff oder gemeinsam mit $R^1$ für Cycloalkyl oder Cycloalkenyl, an die gegebenenfalls ein Benzolring annelliert sein kann, steht.

dadurch gekennzeichnet, daß man Perfluoralkylsulfone der Formel II

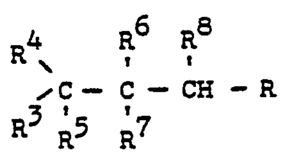

(II)

in welcher

R für den Rest $-SO_2-R_F$,

$R^3$ für Wasserstoff oder Alkyl steht,

$R^4$ für Alkyl steht oder zusammen mit $R^8$ eine Alkendiylbrücke bildet, deren Doppelbindung gegebenenfalls an einen Benzolring annelliert ist,

$R^5$ und $R^6$ entweder für Wasserstoff stehen oder gemeinsam eine Doppelbindung bilden.

$R^7$ für Wasserstoff oder Alkyl steht,

$R^8$ für Wasserstoff oder Alkyl steht und

$R_F$ für Perfluoralkyl steht,

mit der Maßgabe, daß $R^5$ und $R^6$ entweder eine Doppelbindung bilden oder $R^8$ und $R^4$ eine Alkendiylbrücke, deren Doppelbindung gegebenenfalls an einen Benzolring annelliert ist, bilden.

mit β,β-Dimethyl-acrylsäure-derivaten der Formel III

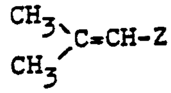

(III)

in welcher

Z die oben angegebene Bedeutung hat,

in Gegenwart von Basen und gegebenenfalls in Gegenwart von Verdünnungsmitteln bei Temperaturen zwischen − 20 und + 30 °C umsetzt.

2. Perfluoralkyl-sulfone der Formel II

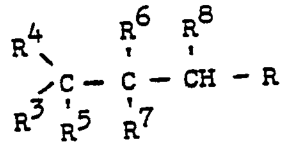

(II)

in welcher

R, $R^3$-$R^8$ die oben angegebene Bedeutung besitzen.

3. Verfahren zur Herstellung von Perfluoralkylsulfonen der Formel II (oben), dadurch gekennzeichnet, daß man Perfluoralkan-sulfinsäuren der Formel IV.

$$R_F—SO_2H \quad (IV)$$

in welcher

$R_F$ für Perfluoralkyl steht,

mit Dienen der Formel V

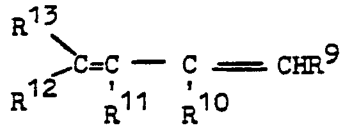

(V)

in welcher

$R^9$-$R^{13}$ für Wasserstoff oder Alkyl stehen, wobei $R^9$ gemeinsam mit $R^{13}$ für eine Alkylenbrücke stehen kann, an die gegebenenfalls ein Cycloalkenring annelliert ist,

gegebenenfalls in Gegenwart von Verdünnungsmitteln bei Temperaturen zwischen − 20 und + 20 °C umsetzt.

**Claims**

1. Process for the preparation of cyclopropane-1-carboxylic acid derivatives of the formula I

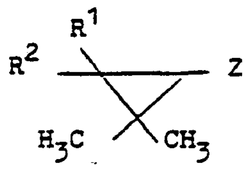

(I)

in which

Z represents alkoxycarbonyl or cyano,

$R^1$ represents hydrogen or alkenyl and

$R^2$ represents hydrogen or, together with $R^1$, cycloalkyl or cycloalkenyl, onto which a benzene ring can optionally be fused.

characterised in that perfluoroalkyl sulphones of the formula II

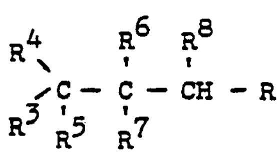

(II)

in which

R represents the radical $—SO_2—R_F$,

$R^3$ represents hydrogen or alkyl,

$R^4$ represents alkyl or, together with $R^8$, forms an alkenediyl bridge, the double bond of which is

optionally fused onto a benzene ring,

$R^5$ and $R^6$ either represent hydrogen or together form a double bond,

$R^7$ represents hydrogen or alkyl,

$R^8$ represents hydrogen or alkyl and

$R_F$ represents perfluoroalkyl,

with the proviso that either $R^5$ and $R^6$ form a double bond or $R^8$ and $R^4$ form an alkenediyl bridge, the double bond of which is optionally fused onto a benzene ring, are reacted with β,β,-dimethyl-acrylic acid derivatives of the formula III

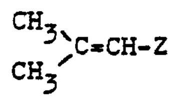

(III)

in which

Z has the meaning indicated above, in the presence of bases and if appropriate in the presence of diluents, at temperatures between − 20 and + 30 °C.

2. Perfluoroalkyl sulphones of the formula II

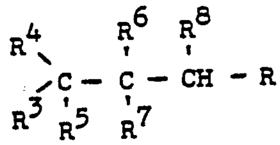

(II)

in which

R and $R^3$-$R^8$ have the meaning indicated above.

3. Process for the preparation of perfluoroalkyl sulphones of the formula II (above), characterised in that perfluoroalkane-sulphinic acids of the formula IV

$$R_F—SO_2H \qquad (IV)$$

in which

$R_F$ represents perfluoroalkyl,

are reacted with dienes of the formula V

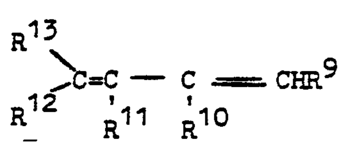

(V)

in which

$R^9$-$R^{13}$ represent hydrogen or alkyl, it being possible for $R^9$, together with $R^{13}$, to represent an alkylene bridge, onto which a cycloalkene ring is optionally fused,

if appropriate in the presence of diluents, at temperatures between − 20 and + 20 °C.

**Revendications**

1. Procédé de préparation de dérivés de l'acide cyclopropane-1-carboxylique de formule I

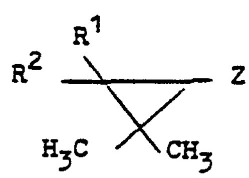

(I)

dans laquelle

Z représente un groupe alcoxycarbonyle ou cyano et

$R^1$ représente l'hydrogène ou un groupe alcényle et

$R^2$ représente l'hydrogène ou forme avec $R^1$ un groupe cycloalkyle ou cycloalcényle sur lequel un cycle benzénique peut, le cas échéant, être condensé,

caractérisé en ce que l'on fait réagir des perfluoralkylsulfones de formule II

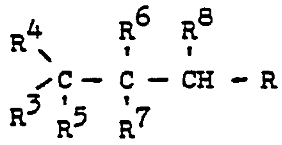

(II)

dans laquelle

R représente le reste — $SO_2$ — $R_F$,

$R_3$ représente l'hydrogène ou un groupe alkyle,

$R^4$ représente un groupe alkyle ou forme avec $R^8$ un pont alcène-diyle dont la double liaison est le cas échéant condensée sur un cycle benzénique,

$R^5$ et $R^6$ représentent des atomes d'hydrogène ou forment en commun une double liaison,

$R^7$ représente l'hydrogène ou un groupe alkyle,

$R^8$ représente l'hydrogène ou un groupe alkyle et

$R_F$ représente un groupe perfluoralkyle,

étant spécifié que ou bien $R^5$ et $R^6$ forment ensemble une double liaison, ou bien $R^8$ et $R^4$ représentent un pont alcène-diyle dont la double liaison est condensée le cas échéant sur un cycle benzénique, avec des dérivés de l'acide bêta, bêta-diméthylacrylique de formule III

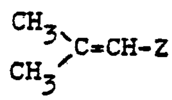

(III)

dans laquelle

Z a les significations indiquées ci-dessus, en présence de bases et le cas échéant en présence de diluants à des températures allant de – 20 à + 30 °C.

2. Perfluoralkylsulfones de formule II

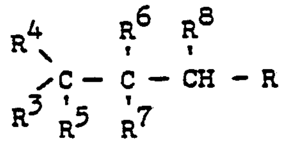

(II)

dans laquelle

R, $R^3$ à $R^8$ ont les significations indiquées ci-dessus.

3. Procédé de préparation des perfluoralkylsulfones de formule II (ci-dessus) caractérisé en ce que l'on fait réagir des acides perfluoralcane-sulfiniques de formule IV

$$RF — SO_2H \qquad (IV)$$

dans laquelle

$R_F$ représente un groupe perfluoralkyle, avec des diènes de formule V

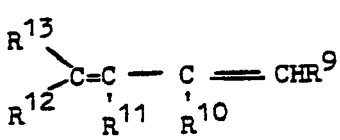

(V)

dans laquelle

$R^9$ à $R^{13}$ représentent l'hydrogène ou des groupes alkyle, $R^9$ pouvant également former avec $R^{13}$ un pont alkylène sur lequel, le cas échéant, un noyau cycloalcène est condensé,

le cas échéant en présence de diluants, à des températures allant de – 20 à + 20 °C.